(19) Europäisches Patentamt
European Patent Office
Office européen des brevets

(11) **EP 4 403 195 A1**

(12) **EUROPEAN PATENT APPLICATION**

(43) Date of publication:
**24.07.2024 Bulletin 2024/30**

(51) International Patent Classification (IPC):
**A61L 27/26** (2006.01)     **A61L 27/52** (2006.01)

(21) Application number: **23152052.9**

(52) Cooperative Patent Classification (CPC):
(C-Sets available)
**A61L 27/26; A61L 27/52;** A61L 2430/06     (Cont.)

(22) Date of filing: **17.01.2023**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Hy2Care B.V.**
**6167 RD Geleen (NL)**

(72) Inventors:
• **Nauta, Laura**
**6167 RD Geleen (NL)**

• **Sen, Kshama Shree**
**6167 RD Geleen (NL)**
• **Breuer, Nick Leonardus Bianca Mathias**
**6167 RD Geleen (NL)**
• **Both, Sanna Karijn**
**6167 RD Geleen (NL)**
• **Karperien, Hermanus Bernardus Johannes**
**6167 RD Geleen (NL)**

(74) Representative: **IPecunia**
**P.O. Box 593**
**6160 AN Geleen (NL)**

(54) **KIT OF PARTS SUITABLE FOR PREPARING A BIOCOMPATIBLE POLYMERIC HYDROGEL**

(57)     The invention relates to a kit of parts suitable for preparing a biocompatible polymeric hydrogel, the kit comprising at least 2 containers each containing an aqueous composition comprising at least one of tyramine-functionalized dextran (Dex-TA), tyramine-functionalized hyaluronic acid (HA-TA), peroxidase and hydrogen peroxide, which components -when mixed- allow the formation of the biocompatible polymeric hydrogel,
wherein each of peroxidase and hydrogen peroxide are present in a different container,
wherein the total weight ratio of Dex-TA and HA-TA is between 75:25 and 25:75, preferably between 60:40 and 40:60, more preferably between 55:45 and 45:55;
wherein the concentration of Dex-TA ranges between 2 and 7 wt% relative to the sum of aqueous compositions of the kit of parts;
wherein the concentration of HA-TA ranges between 2 and 7 wt% relative to the sum of aqueous compositions of the kit of parts;
wherein the DEX-TA comprises unsubstituted and TA substituted repeat units of dextran, and wherein the amount of TA substituted repeat units of DEX-TA ($DS_{DEX}$) is between 5 mol% and 20 mol% relative to the total number of unsubstituted and TA substituted repeat units according to proton NMR;
wherein the HA-TA comprises unsubstituted and TA substituted repeat units of hyaluronic acid, and wherein the amount of TA substituted repeat units of HA-TA ($DS_{HA}$) is between 5 mol% and 20 mol % relative to the total number of unsubstituted and TA substituted repeat units according to proton NMR;
wherein the DEX-TA has a weight average molecular weight (Mw) between 15 and 650 kDa;
wherein the HA-TA has a weight average molecular weight (Mw) between 15 and 550 kDa;
wherein the peroxidase is present in a concentration between 0.25 and 10 Units/ml and the hydrogen peroxide is present in a concentration between 0.01 and 0.10 wt%, relative to the sum of aqueous compositions of the kit of parts.

EP 4 403 195 A1

(52) Cooperative Patent Classification (CPC): (Cont.)

C-Sets
**A61L 27/26, C08L 5/02;**
**A61L 27/26, C08L 5/08**

**Description**

Field of the invention

The invention relates to a kit of parts suitable for preparing a biocompatible polymeric hydrogel, to a process for making a biocompatible polymeric hydrogel, to a mixed polymer composition and to a biocompatible polymeric hydrogel.

The invention further relates to the use of the biocompatible polymeric hydrogel of the invention for repair of damaged cartilage in order to prevent or suppress joints pain in humans or in animals.

Background of the invention

Cartilage is a connective tissue found in many areas of the body (e.g., joints between bones, bronchial tubes, etc.) of higher animals including humans. It is made up of specialized cells called chondrocytes which produce large amounts of extracellular matrix composed of collagen fibers, proteoglycan, and elastin fibers. The self-regeneration of cartilage is low because it does not contain blood vessels (it is avascular) or nerves (it is aneural). Cartilage may be damaged in many ways such as injuries, accidents, and age-related degeneration due to arthritis, in particular osteoarthritis. People with cartilage damage suffer from joint pain, stiffness, decreased range of motion which have impact on daily life. The functional and physical limitation also leads to anxiety and depression. Pain management and total or partial prosthetic joint replacement are the most common therapies in these cases. However, pain management does not solve the underlying cause and only gives temporary relief. Joint replacement on other hand, is usually performed for elderly patients and only at the end stage of the disease. In case of young patients due to the limited lifespan of artificial joints, joint replacement is not recommended. Often, young patients develop osteoarthritis when cartilage damage from sports injuries or accidents are not taken care of early on. Due to these reasons, alternative solutions to joint replacement surgery and repair of cartilage damage early on is important to prevent the onset of joint disease like osteoarthritis. So, preventive treatments of defected joints in human or in animals avoiding the development of osteoarthritis are under investigation.

Although up today, there is still no definitive cure for cartilage defects; tissue engineering is a promising method for the regeneration of degenerated or lost cartilage in human or in animals. Tissue engineering approaches generally involve the use of cells placed in three dimensional scaffolds, the latter acting as a temporary artificial extracellular matrix (ECM). Hydrogels are water-insoluble, three-dimensional networks of polymer chains capable of holding large amounts of water. They are one of the biomaterials designed for use in human body and are therefore used in biomedical applications. A biocompatible polymeric hydrogel may serve as a temporary scaffold to guide chondrocytes and differentiation of chondrocytes and/or their progenitor cells, resulting in newly formed cartilage tissue. The use of a biocompatible polymeric hydrogel has various advantages. It can be prepared in situ from a mixed polymer composition which is locally applied via a minimally invasive surgical procedure. It can fill irregular-shaped defects and allows easy incorporation of cells and bioactive molecules.

Therefore, in recent years, biocompatible polymeric hydrogels have received much attention in cartilage tissue engineering. A mixed polymer composition is typically prepared in which chemical crosslinking reactions are occurring allowing a gelation process which results in the formation of a biocompatible polymeric hydrogel.

Enzymatic crosslinking of dextran-tyramine conjugates (Dex-TA) in the presence of horseradish peroxidase (HRP) and hydrogen peroxide has been disclosed (Jin R. et al., Biomaterials. 2009 (13):2544-51 and Jin R. et al., Biomaterials, 2007 (18):2791-800).

WO 2011/059326 discloses kit of parts suitable for preparing biocompatible polymeric hydrogels comprising heparin-tyramine (Hep-TA) conjugate, dextran-tyramine conjugate (Dex-TA), hydrogen peroxide and peroxidase.

The gelation process and the formation of a suitable polymeric hydrogel is a complex procedure. During polymerization (gelation) of a mixed polymer composition, up to an infinite number of networks are produced. The key issue in this process is bond formation, which in turn causes cluster formation. The formation of more clusters eventually results in gelation. Methods known in the art for preparing a biocompatible polymeric hydrogel have several disadvantages. A fast gelation time of the mixed polymer composition makes it difficult to handle the mixed polymer composition during application because it may create the biocompatible polymeric hydrogel during the injection instead of creating it in situ. A very slow gelation requires long operation times, and therefore would be highly impractical, as well as cost ineffective. Polymeric hydrogels exhibit a relatively large change in volume pertaining to a small change in external stimuli (e.g., temperature), due to the formation of polymeric hydrogel networks, which in turn cause polymer swelling. If the polymeric hydrogel swells too much, it will be dragged out of the application area by movement of the joint. A polymeric hydrogel may have low density in the cross-linked networks and as consequence, the polymeric hydrogel has low storage modulus and is degrading too quickly not allowing a proper cartilage regeneration. Also, a polymeric hydrogel may be susceptible to mechanical erosion between two moving joints.

Prior art kit of parts suitable for preparing a biocompatible polymeric hydrogels and prior art biocompatible polymeric hydrogels are still far from optimal.

The aim of the invention is to overcome at least some of these disadvantages. More specifically, the aim of the invention is to develop an efficient kit of parts suitable for preparing a biocompatible polymeric hydrogel and an efficient biocompatible polymeric hydrogel advantageously used for repair of damaged cartilage in order to prevent or suppress joints pain in humans or in animals.

After extensive studies, the inventors solved at least part of the above-mentioned problems and developed an efficient kit of parts suitable for preparing a biocompatible polymeric hydrogel.

Summary of the invention

The present invention relates to a kit of parts suitable for preparing a biocompatible polymeric hydrogel, the kit comprising at least 2 containers each containing an aqueous composition comprising at least one of tyramine-functionalized dextran (Dex-TA), tyramine-functionalized hyaluronic acid (HA-TA), peroxidase and hydrogen peroxide, which components -when mixed- allow the formation of the biocompatible polymeric hydrogel,

    wherein each of peroxidase and hydrogen peroxide are present in a different container;
    wherein the total weight ratio of Dex-TA and HA-TA is between 75:25 and 25:75, preferably between 60:40 and 40:60, or more preferably between 55:45 and 45:55;
    wherein the concentration of Dex-TA ranges between 2 and 7 wt% relative to the sum of aqueous compositions of the kit of parts;
    wherein the concentration of HA-TA ranges between 2 and 7 wt% relative to the sum of aqueous compositions of the kit of parts;
    wherein the DEX-TA comprises unsubstituted and TA substituted repeat units of dextran, and wherein the amount of TA substituted repeat units of DEX-TA ($DS_{DEX}$) is between 5 mol% and 20 mol% relative to the total number of unsubstituted and TA substituted repeat units according to proton NMR;
    wherein the HA-TA comprises unsubstituted and TA substituted repeat units of hyaluronic acid, and wherein the amount of TA substituted repeat units of HA-TA ($DS_{HA}$) is between 5 mol% and 20 mol % relative to the total number of unsubstituted and TA substituted repeat units according to proton NMR;
    wherein the DEX-TA has a weight average molecular weight (Mw) between 15 and 650 kDa;
    wherein the HA-TA has a weight average molecular weight (Mw) between 15 and 550 kDa;
    wherein the peroxidase is present in a concentration between 0,25 and 10 Units/ml and the hydrogen peroxide is present in a concentration between 0.01 and 0.10 wt%, relative to the sum of aqueous compositions of the kit of parts.

The kit of parts suitable for preparing a biocompatible polymeric hydrogel according to the invention allows cartilage regeneration and may be used for repair of damaged cartilage in order to prevent or suppress joints pain in humans or in animals.

Description of the drawings

    Figure 1 shows the gelation time in seconds of the biocompatible polymeric hydrogel of the invention prepared using 0.03 wt% (dark black color) or 0.05 wt% (light black color) of hydrogen peroxide. The biocompatible polymeric hydrogels were prepared using 3 Units/ml of peroxidase (HRP).
    Figure 2 shows the storage modulus in kPa of the biocompatible polymeric hydrogel of the invention prepared using 0.03 wt% (dark black color) or 0.05 wt% (light black color) of hydrogen peroxide. The biocompatible polymeric hydrogels were prepared using 3 Units/ml of peroxidase (HRP).
    Figure 3 shows the swelling behavior of the biocompatible polymeric hydrogel of the invention prepared using 0.03 wt% (dark black color) or 0.05 wt% (light black color) of hydrogen peroxide. The biocompatible polymeric hydrogels were prepared using 3 Units/ml of peroxidase (HRP).
    Figure 4 shows the degradation over time of the biocompatible polymeric hydrogel of the invention prepared using 0.03 wt% (dark black color) or 0.05 wt% (light black color) of hydrogen peroxide. The biocompatible polymeric hydrogels were prepared using 3 Units/ml of peroxidase (HRP).
    Figure 5 shows the mold sheet used to evaluate the storage modulus and the swelling behavior of the biocompatible polymeric hydrogel of the invention.

## Detailed description of the invention

The present invention relates to a kit of parts suitable for preparing a biocompatible polymeric hydrogel, the kit comprising at least 2 containers each containing an aqueous composition comprising at least one of tyramine-functionalized dextran (Dex-TA), tyramine-functionalized hyaluronic acid (HA-TA), peroxidase and hydrogen peroxide, which components -when mixed- allow the formation of the biocompatible polymeric hydrogel,

wherein each of peroxidase and hydrogen peroxide are present in a different container;
wherein the total weight ratio of Dex-TA and HA-TA is between 75:25 and 25:75, preferably between 60:40 and 40:60, more preferably between 55:45 and 45:55;
wherein the concentration of Dex-TA ranges between 2 and 7 wt% relative to the sum of aqueous compositions of the kit of parts;
wherein the concentration of HA-TA ranges between 2 and 7 wt% relative to the sum of aqueous compositions of the kit of parts;
wherein the DEX-TA comprises unsubstituted and TA substituted repeat units of dextran, and wherein the amount of TA substituted repeat units of DEX-TA ($DS_{DEX}$) is between 5 mol% and 20 mol% relative to the total number of unsubstituted and TA substituted repeat units according to proton NMR;
wherein the HA-TA comprises unsubstituted and TA substituted repeat units of hyaluronic acid, and wherein the amount of TA substituted repeat units of HA-TA ($DS_{HA}$) is between 5 mol% and 20 mol % relative to the total number of unsubstituted and TA substituted repeat units according to proton NMR;
wherein the DEX-TA has a weight average molecular weight (Mw) between 15 and 650 kDa;
wherein the HA-TA has a weight average molecular weight (Mw) between 15 and 550 kDa;
wherein the peroxidase is present in a concentration between 0,25 and 10 Units/ml and the hydrogen peroxide is present in a concentration between 0.01 and 0.10 wt%, relative to the sum of aqueous compositions of the kit of parts.

When tyramine-functionalized dextran (Dex-TA), tyramine-functionalized hyaluronic acid (HA-TA), peroxidase and hydrogen peroxide as above described are mixed, a mixed polymer composition is obtained. A gelation process will start within the mixed polymer composition providing a biocompatible polymeric hydrogel.

## Detailed description of the features

The term "polymeric hydrogel" as used herein refers to a three-dimensional hydrophilic polymeric network. The polymeric hydrogel of the current invention has high water content, providing an environment similar to native cartilage. Moreover, the polymeric hydrogel of the current invention allows for sufficient transportation of nutrients and waste products, which is essential for cell growth. Preferably, the polymeric hydrogel of the current invention is obtained in-situ when injecting the mixed polymer composition as above described.

The kit of parts of the current invention and the mixed polymer composition of the current invention comprise polymers or functionalized polymers which may be selected from natural polymers, polymers present in cartilage tissue or modified natural polymers.

Polymers to be used in the current invention may be gelatin (amide based), collagen, polysaccharides, such as Dextran, and glycosaminoglycans (GAGs), including hyaluronic acid, chondroitin sulphate, keratin sulphate, heparin sulphate and chitosans.

Preferably, the polymers used in the current invention are based on dextran and hyaluronic acid which are biocompatible, and easily modifiable being therefore a perfect template material for further functionalization.

The term "biocompatible polymeric hydrogel" as used herein refers to a polymeric hydrogel that has the ability to be in contact with a living system without producing a significant adverse effect. Biocompatible in this context means that a material does not elicit a significant pathological response of the body against said biomaterial or that said material is not harmful to the patient. Standard ISO tests may be used to evaluate the biocompatibility of a polymeric hydrogel, particularly considering problems of cytotoxicity, irritation, sensitization, acute or sub-acute or sub chronic systemic toxicity, pyrogenicity and genotoxicity.

The kit of parts suitable for preparing a biocompatible polymeric hydrogel of the current invention comprises at least 2 containers.

The term "container" as used herein refers to an object for holding or transporting an aqueous composition. Any type of containers suitable for holding or transporting substances may be used. Preferably, the container is suitable for holding or transporting medical substances or substances as tyramine-functionalized dextran (Dex-TA), tyramine-functionalized hyaluronic acid (HA-TA), peroxidase and hydrogen peroxide. Preferably, the containers used in the kit of parts of the current invention are assembled in an applicator device.

Each container contains an aqueous composition comprising at least one of tyramine-functionalized dextran (Dex-TA), tyramine-functionalized hyaluronic acid (HA-TA), peroxidase and hydrogen peroxide while having peroxidase and hydrogen peroxide in a different container avoiding the gelation process and the formation of the polymeric hydrogel in the applicator device.

A tyramine-functionalized dextran (Dex-TA) and a tyramine-functionalized hyaluronic acid (HA-TA) are prepared by modifying dextran and hyaluronic acid by a coupling reaction with tyramine (TA) following a procedure known in art (Dex-TA as reported by R Jin et al. Journal of Controlled Release 2008;132: e24-e6, and Dex-TA conjugate and HA-TA conjugate as disclosed in WO 2011/059326 and in WO 2019/143247), both incorporated herein by reference.

Tyramine (TA) has been selected as reactive side group that can be enzymatically crosslinked. Tyramine-functionalized dextran (Dex-TA) and tyramine-functionalized hyaluronic acid (HA-TA) are crosslinked in situ originating in a biocompatible polymeric hydrogel thanks to the formation of tyramine-tyramine bonds in the presence of a catalyst such as horseradish peroxidase (HRP) and in the presence of an oxidizer such as hydrogen peroxide ($H_2O_2$).

The term "tyramine-functionalized dextran (Dex-TA)" as used herein refers to a dextran molecule conjugated with tyramine molecules linked by a urethane bond or by any bonds allowing the conjugation of the dextran molecule with Tyramine.

The term "tyramine-functionalized hyaluronic acid (HA-TA)" as used herein refers to a hyaluronic acid molecule conjugate with tyramine molecules linked by an amide bond or by any bonds allowing the conjugation of the hyaluronic acid molecule with Tyramine.

The functional properties of the biocompatible polymeric hydrogel of the current invention are influenced from many factors. Important properties are the shear storage modulus, the swell, the shrinkage, glucose permeability and the degradation of the biocompatible polymeric hydrogel.

The term "shear storage modulus" as used herein, refers to a parameter calculated from the strain and stress measurement values obtained when the biocompatible polymeric hydrogel has been subjected to an experiment performed in shear plate-plate geometry. The storage modulus relates to the biocompatible polymeric hydrogel ability to store energy elastically (energy stored in the elastic structure of the biocompatible polymeric hydrogel). Preferably, the storage modulus is high such that the biocompatible polymeric hydrogel is a firm gel to be able to withstand all forces potentially encountered in a joint. Preferably, the storage modulus is not too high allowing cell penetration and proliferation in the biocompatible polymeric hydrogel.

The term "swell" as used herein, refers to the increase of volume of the biocompatible polymeric hydrogel after its formation in situ. The swell of a biocompatible polymeric hydrogel which is a porous fluid-saturated material may be triggered by various stimuli. The swell induces deformation of the biocompatible polymeric hydrogel which may be pushed out from the injected area (e.g. joint) during movement and causing removal of the hydrogel from the defected area.

The term "shrinkage" as used herein refers to the volume reduction of the mixed polymer composition transformed in biocompatible polymeric hydrogel. Preferably, the biocompatible polymeric hydrogel has a small shrinkage such that to prevent the bulging of the biocompatible polymeric hydrogel out of the injected area during movement and the detachment of the biocompatible polymeric hydrogel from the defected tissue, causing removal of the hydrogel from the defected area.

The term "degradation" as used herein refers to the loss of mass of the biocompatible polymeric hydrogel sample, upon exposure to a 5 U/mL hyaluronidase solution at 37°C. Preferably, the degradation is not very fast to provide enough time for chondrocytes to migrate in the biocompatible polymeric hydrogel and produce extracellular matrix. In a preferred embodiment the degradation is less than 50 wt% in the first 30 days after formation of the hydrogel.

A first factor influencing the functional properties of the biocompatible polymeric hydrogel of the current invention, is the weight ratio of Dex-TA and HA-TA.

The inventor found that preparing a biocompatible polymeric hydrogel using only HA-TA results in a biocompatible polymeric hydrogel having poor mechanical properties. If only Dex-TA is used, no in-situ degradation will occur. The inventors found instead that acceptable functional properties of the biocompatible polymeric hydrogel of the current invention could be obtained when Dex-TA and HA-TA are used in the kit of parts of the current invention in a total weight ratio of between 75:25 and 25:75, preferably between 60:40 and 40:60, most preferably between 55:45 and 45:55.

The inventors of the present invention observed that the storage modulus improves when the weight ratio of Dex-TA/HA-TA is between 75:25 and 25:75, preferably between 60:40 and 40:60, more preferably when the weight ratio of Dex-TA/HA-TA is between 55:45 and 45:55.

Regarding the effect of the weight ratio of Dex-TA/HA-TA on the degradation of the biocompatible polymeric hydrogel of the current invention, the inventors noticed that the degradation is slower when Dex-TA/HA-TA is between 75:25 and 25:75, preferably between 60:40 and 40:60, more preferably when the weight ratio of Dex-TA/HA-TA is between 55:45 and 45:55.

A second factor is the concentration of Dex-TA and HA-TA relative to the sum of aqueous compositions of the kit of parts. The inventors found that acceptable functional properties of the biocompatible polymeric hydrogel of the current invention could be obtained when the concentration of Dex-TA ranges between 2 and 7 wt%, preferably between 3 and 6 wt%, more preferably between 3 and 5 wt%, relative to the sum of aqueous compositions of the kit of parts and when the concentration of HA-TA ranges between 2 and 7 wt%, preferably between 3 and 6 wt%, more preferably between 3 and 5 wt%, relative to the sum of aqueous compositions of the kit of parts. The inventors of the present invention observed that if the concentration of Dex-TA and HA-TA is lower than 2 wt% the storage modulus of the biocompatible polymeric hydrogel decreases, and the biocompatible polymeric hydrogel cannot withstand the forces in the injected area (e.g. a joint) and it can be dragged out from the injected area (e.g. a joint) by a movement.

The concentration of each of DEX-TA and HA-TA preferably ranges between 2-10 wt% in a container, when DEX-TA and/or HA-TA are present in the container.

A third factor is the degree of substitution (DS) of tyramine on the dextran and on the hyaluronic acid. The term "degree of substitution (DS)" with respect to dextran and to hyaluronic acid, refers to the (average) number of substituent (TA) groups attached per repeat unit considering that the repeat unit for dextran is a monosaccharide while the repeat unit for hyaluronic acid is a disaccharide.

The inventor found that acceptable functional properties of the biocompatible polymeric hydrogel of the current invention could be obtained when DEX-TA comprises unsubstituted and TA substituted repeat unit of dextran wherein the amount of TA substituted repeat units of DEX-TA ($DS_{DEX}$) is between 5 mol% and 20 mol%, preferably between 6 % and 17 %, more preferably between 7 % and 15 %, even more preferably between 7.5 % and 12 %, even further more preferably between 7.5 % and 10.5 % relative to the total number of unsubstituted and TA substituted repeat units according to proton NMR. Slightly slower degradation of the biocompatible polymeric hydrogel has been observed when $DS_{DEX}$ is lower than 5 mol% while $DS_{DEX}$ higher than 12 mol% makes the formation of the biocompatible polymeric hydrogel being very slow.

The inventor found that acceptable functional properties could be obtained when HA-TA comprises unsubstituted and TA substituted repeat units of hyaluronic acid wherein the amount of TA substituted -repeat units of HA-TA ($DS_{HA}$) is between 5 mol% and 20 mol %, preferably between 6 % and 17 %, more preferably between 7 % and 15 %, even more preferably between 10 % and 14.5 % relative to the total number of unsubstituted and TA substituted repeat units according to proton NMR.

A fourth factor is the weight average molecular weight (Mw) of DEX-TA and HA-TA. The inventor found that acceptable functional properties of the biocompatible polymeric hydrogel of the current invention could be obtained when DEX-TA has a weight average molecular weight (Mw) between 15 and 650 kDa, preferably between 20 and 125, more preferably between 30 and 60 and when the HA-TA has a weight average molecular weight (Mw) between 15 and 550 kDa. preferably between 15 and 300, more preferably between 15 and 60.

Also, the preparation process of the biocompatible polymeric hydrogel is influenced from many factors. In particular, the viscosity, injectability and the gelation time of the mixed polymer composition should be considered to obtain an efficient process.

The term "injectability" as used herein indicates that the mixed polymer composition can be introduced into the body using an injection applicator device which may have a needle.

The term "gelation time" as used herein refers to the time required for the cross-linked polymer to change from a free-flowing solution to a gel or semi-gel state.

A first factor to be considered in the preparation of the biocompatible polymeric hydrogel of the current invention is the quantity of the peroxidase. The inventor found that an efficient process for obtaining the biocompatible polymeric hydrogel of the current invention is achieved when the peroxidase is used in a concentration between 0.25 and 10 Units/ml relative to the sum of aqueous compositions of the kit of parts. Preferably, when the peroxidase is used in a concentration between 1 and 5 Units/ml, more preferably between 2 and 4 Units/ml, even more preferably between 2 and 3.5 Units/ml relative to the sum of aqueous compositions of the kit of parts.

The term "Units/ml" in relation to the peroxidase also known as "pyrogallol units/ml" refers to the concentration of peroxidase expressed in "active units" related to the enzymatic activity which is what controls the formation of the biocompatible polymeric hydrogel. Specifically, it refers to the amount of enzyme that forms 1.0 mg of purpurogallin from pyrogallol in 20s at pH 6.0 and 20°C, as determined with a spectrophotometric assay.

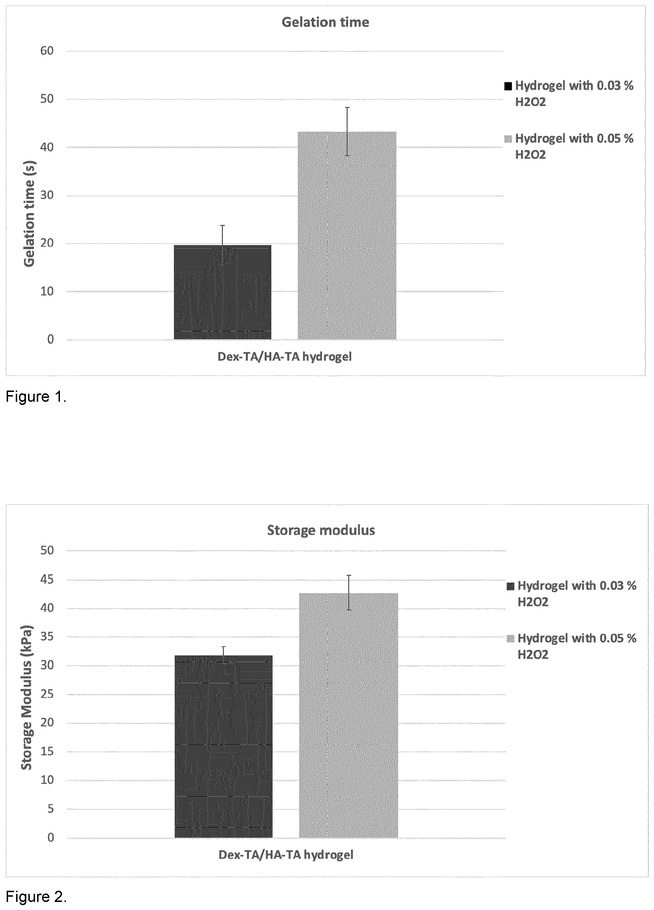

Regarding the effect of the quantity of the peroxidase on the gelation time of the mixed polymer composition of the invention, the inventors monitored the gelation of the mixed polymer composition and observed that decreasing the quantity of the peroxidase increased the gelation time improving the handleability and usability of the kit of parts of the current invention. The optimal gelation time which is in a range between 35 and 60 seconds was obtained when using the peroxidase in a concentration of 3 Units/ml relative to the sum of aqueous compositions of the kit of parts as indicated in Figure 1. This gelation time improves the handleability and usability of the kit of parts of the current invention.

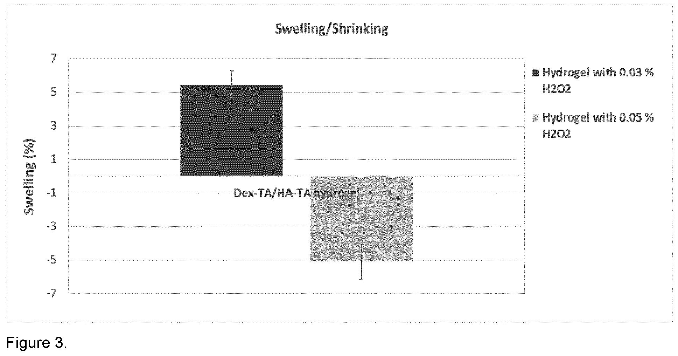

The quantity of the peroxidase has also effect on the storage modulus of the biocompatible polymeric hydrogel of the invention as indicated in Figure 2 and on swelling/shrinkage of the biocompatible polymeric hydrogel of the invention as indicated in Figure 3. The optimal storage modulus, being in a range between 30 and 60 kPa, and can be obtained when using the peroxidase in a concentration of 3 Units/ml relative to the sum of aqueous compositions of the kit of parts. Also, swelling is avoided and optimal shrinkage, which is in a range between 0% and 10%, can be obtained when using the peroxidase in a concentration of 3 Units/ml relative to the sum of aqueous compositions of the kit of parts

Any peroxidase known by the skilled person and suitable for the preparation of a biocompatible polymeric hydrogel may be used. Preferably the peroxidase to be used in the current invention is selected from horseradish peroxidase (HRP), soybean peroxidase, myeloperoxidase, lactoperoxidase, Nitrospira sp. recombinant and Peroxidase and Lyngbya sp. recombinant lysyl oxidase. The preferred peroxidase to be used in the current invention is horseradish peroxidase (HRP).

A second factor is the quantity of hydrogen peroxide. The inventor found that an efficient process for obtaining the biocompatible polymeric hydrogel of the current invention is achieved when hydrogen peroxide is used in a concentration between 0.01 and 0.10 wt% relative to the sum of aqueous compositions of the kit of parts. Preferably between 0.02 and 0.07 wt%, more preferably between 0.025 and 0.06 wt%, even more preferably between 0.03 and 0.055 wt% relative to the sum of aqueous compositions of the kit of parts.

Regarding the effect of the quantity of hydrogen peroxide on the gelation time of the mixed polymer composition, the inventors found that when using the hydrogen peroxide in a concentration of 0.17 wt% relative to the sum of aqueous compositions of the kit of parts; the mixed polymer composition would gelate in 13 minutes. Lowering the concentration of hydrogen peroxide to 0.1 wt% relative to the sum of aqueous compositions of the kit of parts would provide a gelation in 197 seconds. Lowering further the concentration of hydrogen peroxide to 0.01 wt% relative to the sum of aqueous compositions of the kit of parts, would provide a gelation in 14 seconds. The optimal gelation time which improves the handleability and usability of the kit of parts of the current invention, is in a range between 35 and 60 seconds and it was obtained when using the hydrogen peroxide in a concentration of 0.05 wt% relative to the sum of aqueous compositions of the kit of parts (as indicated in Figure 1).

Additionally, when hydrogen peroxide is used at 0.05 wt% optimal storage moduli and shrinkage/swelling of the biocompatible polymeric hydrogel of the invention as indicated in Figure 2 and 3 is obtained. The optimal storage modulus which is in a range between 30 and 60 kPa, is such that the biocompatible polymeric hydrogel can withstand the forces in the injected area (e.g. a joint) but is soft enough for cells to migrate into the biocompatible polymeric hydrogel and proliferate. Also, swelling is avoided and optimal shrinkage which is in a range between 0% and 10% was obtained when using hydrogen peroxide at 0.05 wt% relative to the sum of aqueous compositions of the kit of parts.

Hence, when hydrogen peroxide is used at 0.05 wt% the biocompatible polymeric hydrogel has a suitable stiffness, and it is therefore less susceptible to mechanical erosion between two moving joints, and it shows to provide an optimal density of the biocompatible polymeric hydrogel allowing chondrocytes migration and growing into the biocompatible polymeric hydrogel and therefore allowing for the cartilage regeneration.

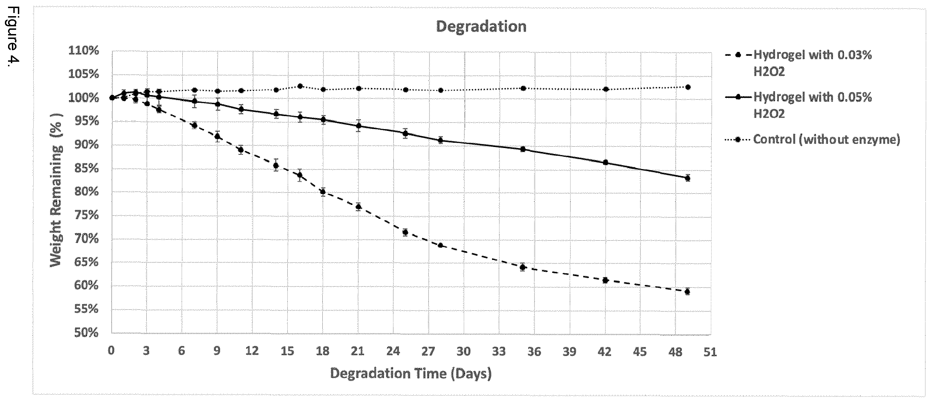

Finally, when hydrogen peroxide is used at 0.05 wt% an optimal biocompatible polymeric hydrogel is consistently obtained, also characterized by a slow degradation as shown in Figure 4, which gives time to the chondrocytes to migrate and grow into the biocompatible polymeric hydrogel.

From the above, the inventors found that both the quantity of peroxidase and of hydrogen peroxide are influencing the gelation time of the mixed polymer composition, the storage modulus and shrinkage/swelling (measured as gap size) of the biocompatible polymeric hydrogel. Therefore, the assessment of both their effect was conducted at a fixed polymer concentration of 8.9 wt% (4.45 wt% of Dex-TA + 4.45 wt% of HA-TA) at a temperature of 18-22 °C for the gelation measurement, at 20±0.1 °C for the storage modulus and gap size measurement considering various concentration combinations of peroxidase (HRP) and hydrogen peroxide as indicated in Table 1.

H2O2 concentration

**Storage modulus (kPa)**

| | 0.017 wt% | 0.028 wt% | 0.040 wt% | 0.053 wt% | 0.079 wt% |
|---|---|---|---|---|---|
| **2.1 U/mL** | | | | 39,4 | 14,1 |
| **3.0 U/mL** | 12,4 | 29,0 | 37,5 | 38,0 | 45,8 |
| **4.2 U/mL** | 13,5 | | | 39,7 | |

H2O2 concentration

**Gelation time (s)**

| | 0.017 wt% | 0.028 wt% | 0.040 wt% | 0.053 wt% | 0.079 wt% |
|---|---|---|---|---|---|
| **2.1 U/mL** | | | | 91,5 | 195,3 |
| **3.0 U/mL** | 15,3 | 21,0 | 36,0 | 54,3 | 57,3 |
| **4.2 U/mL** | 10,7 | | | 32,7 | |

HRP concentration

| Swelling / Shrinking (%) | H2O2 concentration | | | | |
|---|---|---|---|---|---|
| HRP concentration | 0.017 wt% | 0.028 wt% | 0.040 wt% | 0.053 wt% | 0.079 wt% |
| 2.1 U/mL | | | | -1,8% | -9,0% |
| 3.0 U/mL | 15,7% | 4,4% | -1,0% | -5,6% | -4,0% |
| 4.2 U/mL | 15,9% | | | -4,3% | |

Table 1 shows that the combination of high hydrogen peroxide concentrations (extreme maximum) and low peroxidase (HRP) concentration (extreme minimum) leads to very slow gelation, which would require long operation times, and therefore would be highly impractical, as well as cost ineffective. The combination of low hydrogen peroxide (extreme minimum) and high peroxidase (HRP) concentration (extreme maximum) leads instead to very fast gelation, decreasing handleability and convenience for the use of the kit of parts of the invention.

Further, a low hydrogen peroxide concentration (extreme minimum) leads to formation of less densely cross-linked networks, which is reflected both by the low storage moduli and the high degrees of swelling, which could allow gels to be dragged out from the injected area (e.g., a joint) by a movement.

Moreover, the obtained biocompatible polymeric hydrogel of the invention showed to have a relatively low storage modulus compared to prior art biomaterials and biocompatible polymeric hydrogel and to be less susceptible to be dragged out or removed mechanically between two moving joints as it did not swell, and it had a good density for structural support. Also, the degradation time of the polymeric hydrogel was slower allowing for sufficient structural support in the defect so that more time is given to the chondrocytes to migrate and grow allowing for the new cartilage regeneration. Hence, the kit of parts of the current invention allows to obtain an advantageous biocompatible hydrogel/cell constructs when the peroxidase is present in a concentration between 0.25 and 10 Units/ml and the hydrogen peroxide is present in a concentration between 0.01 and 0.10 wt% relative to the sum of aqueous compositions of the kit of parts. Preferably, when the peroxidase is present in a concentration between 1 and 5 Units/ml and the hydrogen peroxide is present in a concentration between 0.02 and 0.07 wt%, more preferably when the peroxidase is present in a concentration between 2 and 4 Units/ml and the hydrogen peroxide is present in a concentration between 0.025 and 0.06 wt%, even more preferably when the peroxidase is present in a concentration between 2 and 3.5 Units/m and the hydrogen peroxide is present in a concentration between 0.03 and 0.055 wt% relative to the sum of aqueous compositions of the kit of parts.

The kit of parts of the current invention as described above is such that the aqueous composition of each container comprises between 80 and 99.9 wt%, preferably between 83 and 99.8 wt%, more preferably between 86 and 99.8 wt%, most preferably between 89 and 99.8 wt% of water, a water saline solution, a phosphate-buffered solution, or a phosphate-buffered saline solution.

The term "water saline solution" as used herein refers to a saline solution (also known as saline) which is a mixture of sodium chloride (salt) and water, and it is commonly used in medicine.

The term "phosphate-buffered solution" also known as PBS as used herein refers to a buffer solution having a pH around 7.4.

The term "phosphate-buffered saline solution" as used herein refers to a water-based salt solution having a pH around 7.4 containing salts as disodium hydrogen phosphate, sodium chloride, potassium chloride or potassium dihydrogen phosphate. The buffer helps to maintain a constant pH and the osmolarity and ion concentrations of the solutions match those of the human body (isotonic).

Water or of a water saline solution or of a phosphate-buffered solution, or of a phosphate-buffered saline solution are commonly used in medicine.

Typically, Dex-TA and HA-TA may be added to water, a water saline solution, a phosphate-buffered solution or a phosphate-buffered saline solution (preferably to a phosphate-buffered saline solution with pH around 7.4) to obtain a polymer solution. The peroxidase may also be dissolved in water, a water saline solution, a phosphate-buffered solution or a phosphate-buffered saline solution to obtain a desired concentration of peroxidase. A solution of hydrogen peroxide may be prepared, preferably freshly prepared, in water, a water saline solution, a phosphate-buffered solution or in a phosphate-buffered saline solution to obtain a hydrogen peroxide solution having the desired concentration.

One preferred embodiment of the current invention relates to a kit of parts comprising a first container which comprises an aqueous composition A, which composition A comprises

a. tyramine-functionalized dextran (Dex-TA),
b. tyramine-functionalized hyaluronic acid (HA-TA),
c. peroxidase,

and wherein a second container comprises an aqueous composition B, which composition B comprises

d. hydrogen peroxide and
e. optionally at least one of tyramine-functionalized dextran (Dex-TA) and tyramine-functionalized hyaluronic acid (HA-TA), preferably both Dex-TA and HA-TA,

wherein the total weight ratio of Dex-TA and HA-TA is between 75:25 and 25:75, preferably between 60:40 and 40:60, more preferably between 55:45 and 45:55;
wherein the concentration of Dex-TA ranges between 2 and 7 wt% relative to the sum of aqueous compositions of the kit of parts;
wherein the concentration of HA-TA ranges between 2 and 7 wt% relative to the sum of aqueous compositions of the kit of parts;
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR;
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR;
wherein the DEX-TA has a weight average molecular weight (Mw) between 15 and 650 kDa;
wherein the HA-TA has a weight average molecular weight (Mw) between 15 and 550 kDa;
wherein the peroxidase is present in a concentration between 0.25 and 10 Units/ml and the hydrogen peroxide is present in a concentration between 0.01 and 0.10 wt%, relative to the sum of aqueous compositions of the kit of parts.

The detailed description of the features as provided for the invention as described directly under the header "Detailed description of the features" equally applies to this preferred embodiment unless differently specified in the text below.

In this specific embodiment the kit of parts of the current invention comprises two containers, a first container which comprises an aqueous composition A as defined above and a second container which comprises an aqueous composition B as defined above.

The volume-to-volume ratio of composition A to composition B is in the range of 0.1 :1 to 10:1, preferably in the range of 0.5:1 to 7:1, more preferably in the range of 0.8:1 to 5:1.

The components A and B of the kit of parts as above described, are both fluids in a temperature range of 0°C to 37°C, having a viscosity between 1 mPa.s and 50 Pa.s, preferably between 1 and 100 mPa.s, more preferably below 50 mPa.s, even more preferably below 35 mPa.s as determined in a rheometer in a double gap (for low viscosity) or cone-plate setup (for higher viscosity) as required at 25±0.1 °C at a shear rate of 10 Hz.

The term "fluids" as used herein refers to a substance whose molecules move freely past one another, such as a liquid, that can flow, has no fixed shape and conform to the shape of their containers and offers little resistance to an external stress.

The kit of parts of this specific embodiment of the current invention as described above is such that each of the composition A or composition B comprises between 80 and 99.9 wt%, preferably between 83 and 99.8 wt%, more preferably between 86 and 99.8 wt%, most preferably between 89 and 99.8 wt% of water, a water saline solution, a phosphate-buffered solution or phosphate-buffered saline solution.

The term "water saline solution", "phosphate-buffered solution" and "phosphate-buffered saline solution" are the same as defined in the description above. The preparation of Dex-TA, HA-TA, hydrogen peroxide and peroxidase (e.g., HRP) solution with water or of a water saline solution or of a phosphate-buffered solution, or of a phosphate-buffered saline solution, is the same as described in the description above.

In a specific embodiment, composition A is prepared mixing in one container the Dex-TA, the HA-TA and the peroxidase solution in phosphate-buffered saline solution while composition B is prepared having in the second container only the hydrogen peroxide solution in phosphate-buffered saline solution or mixing the hydrogen peroxide solution in phosphate-buffered saline solution with Dex-TA, the HA-TA solution in phosphate-buffered saline solution.

Any type of assembled kit as known to workers skilled in the art, suitable for preparing a biocompatible polymeric hydrogel and comprising at least 2 containers, may be used for the current invention. Preferably the outlet of the containers are led to a mixing element, such as a static mixer, to effectively mix the contents of the different containers.

Preferably, the kit of parts of the current invention is an injection kit. Common commercially known injection kit may be used as the Nordson Medical Applicator Assembly device or Sulzer Medmix K-system.

Preferably, the kit comprises separate outlets for each container to reduce the risk of cross-contamination during the application process. Preferably, the content of each container is injected simultaneously, and preferably mixed before leaving the kit. In a preferred embodiment, said kit of parts suitable for preparing a biocompatible polymeric hydrogel is a syringe. The kit of parts suitable for preparing a biocompatible polymeric hydrogel according to the invention may also be marketed as pre-filled syringes.

Using the kit of parts of the current invention as described above to a surgical site or to a site of an incision, allows for reparation of damaged cartilage in order to prevent or suppress joints pain in humans or in animals.

The administration by injection enables in situ micro- and macro-gel formation at the injection site such as the joint space. The surgery to a site such as the joint space may be performed by an incision as an open surgery or by arthroscopic surgery; an incision is made according to the size and location of the defected area. Often a retractor is used to gain the visual of the defected area. Any rough and loose defected cartilage is removed, and the size and depth of the defected area is determined. The defected area is carefully debrided to remove any fissure or degenerated cartilage. The defected area is then positioned horizontally and is made sure that the defected area is dry. The biocompatible polymeric hydrogel is then injected carefully to fill until the edge of the defected area. After few minutes the stability of the biocompatible polymeric hydrogel is checked by a soft movement of the area of application, for example by bending a knee if the biocompatible polymeric hydrogel was applied into the knee. Then the incision is closed following standard procedure.

The current invention relates also to a process for making a biocompatible polymeric hydrogel comprising the steps of:

a. mixing aqueous compositions comprising tyramine-functionalized dextran (Dex-TA), tyramine-functionalized hyaluronic acid (HA-TA), peroxidase and hydrogen peroxide, to obtain a mixed polymer composition,
b. allowing the mixed polymer composition to form the biocompatible polymeric hydrogel during a time between 10 and 200 seconds at a temperature between 15 to 38 °C,

wherein the total weight ratio of Dex-TA and HA-TA is between 75:25 and 25:75, preferably between 60:40 and 40:60, more preferably between 55:45 and 45:55;
wherein the concentration of Dex-TA ranges between 2 and 7 wt% relative to the mixed polymer composition,
wherein the concentration of HA-TA ranges between 2 and 7 wt% relative to the mixed polymer composition,
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the DEX-TA has a weight average molecular weight (Mw) between 15 and 650 kDa,
wherein the HA-TA has a weight average molecular weight (Mw) between 15 and 550 kDa,
wherein the peroxidase is present in a concentration between 0,25 and 10 Units/ml, and the hydrogen peroxide is present in a concentration between 0.01 and 0.10 wt% relative to the mixed polymer composition.

The detailed description of the features as provided for the invention as described directly under the header "Detailed description of the features" equally applies to this embodiment unless differently specified in the text below.

The process for making a biocompatible polymeric hydrogel involves mixing the aqueous compositions comprising tyramine-functionalized dextran (Dex-TA), tyramine-functionalized hyaluronic acid (HA-TA), peroxidase and hydrogen peroxide, to obtain a mixed polymer composition. The mixing may be performed by a procedure known in the art. Preferably, the mixing is performed by using a blending connector or a cannula with a static mixer which may be connected to the applicator device. The inventors found that using the concentration of Dex-TA and Ha-TA as indicated above, together with the specific weight ratio of Dex-TA and HA-TA, with the specific $DS_{DEX}$ and $DS_{HA}$, with the specific DEX-TA and HA-TA weight average molecular weight, with the specific peroxidase and hydrogen peroxide concentration as indicated above; allow the formation of the biocompatible polymeric hydrogel during a time between 10 and 200 seconds, preferably between 20 and 100 seconds, more preferably between 30 and 60 seconds at a temperature between 15 to 38 °C. Specifically, to a temperature between 35 to 38 °C when the process is performed in vivo and to a temperature between 18 to 22 °C, preferably between 17 to 20 °C when the process is performed in vitro.

In a preferred embodiment, the current invention relates to a process for making a biocompatible polymeric hydrogel comprising the steps of:

a. mixing an aqueous composition A and an aqueous composition B obtaining a mixed polymer composition
b. allowing the mixed polymer composition to form the biocompatible polymeric hydrogel during a time between 10 and 200 seconds at a temperature between 15 to 38 °C,

wherein the aqueous composition A comprises tyramine-functionalized dextran (Dex-TA), tyramine-functionalized hyaluronic acid (HA-TA),peroxidase, and wherein the aqueous composition B comprises hydrogen peroxide and optionally at least one of tyramine-functionalized dextran (Dex-TA) and tyramine-functionalized hyaluronic acid (HA-TA), preferably both Dex-TA and HA-TA,

wherein the total weight ratio of Dex-TA and HA-TA is between 75:25 and 25:75, preferably between 60:40 and 40:60, more preferably between 55:45 and 45:55; wherein the concentration of Dex-TA ranges between 2 and 7 wt% relative to the sum of compositions A and B,

wherein the concentration of HA-TA ranges between 2 and 7 wt% relative to the sum of compositions A and B,
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the DEX-TA has a weight average molecular weight (Mw) between 15 and 650 kDa,
wherein the HA-TA has a weight average molecular weight (Mw) between 15 and 550 kDa,
wherein the peroxidase is present in a concentration between 0.25 and 10 Units/ml, and the hydrogen peroxide is present in a concentration between 0.01 and 0.10 wt% relative to the sum of aqueous compositions A and B.

The detailed description of the features as provided for the invention as described directly under the header "Detailed description of the features" equally applies to this preferred embodiment unless differently specified in the text below.

The current invention relates also to a mixed polymer composition obtainable by mixing aqueous compositions comprising tyramine-functionalized dextran (Dex-TA), tyramine-functionalized hyaluronic acid (HA-TA), peroxidase and hydrogen peroxide, wherein the total weight ratio of Dex-TA and HA-TA is between 75:25 and 25:75, preferably between 60:40 and 40:60, more preferably between 55:45 and 45:55;

wherein the concentration of Dex-TA ranges between 2 and 7 wt% relative to the mixed polymer composition,
wherein the concentration of HA-TA ranges between 2 and 7 wt% relative to the mixed polymer composition,
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the DEX-TA has a weight average molecular weight (Mw) between 15 and 650 kDa,
wherein the HA-TA has a weight average molecular weight (Mw) between 15 and 550 kDa,
wherein the peroxidase is present in a concentration between 0.25 and 10 Units/ml, and the hydrogen peroxide is present in a concentration between 0.01 and 0.10 wt% relative to the mixed polymer composition.

The detailed description of the features as provided for the invention as described directly under the header "Detailed description of the features" equally applies to this preferred embodiment unless differently specified in the text below.

The inventor found that if the mixed polymer composition gelates too quickly it will not allow an easy application at the surgical site or to a site of an incision. The inventor found that optimal gelation time of the mixed polymer composition of the current invention could be obtained when the concentration of Dex-TA and HA-TA as indicated above, together with the specific weight ratio of Dex-TA and HA-TA, with the specific $DS_{DEX}$ and $DS_{HA}$, with the specific DEX-TA and HA-TA weight average molecular weight, with the specific peroxidase and hydrogen peroxide concentration as indicated above; allow the gelation of the mixed polymer composition and therefore the formation of the biocompatible polymeric hydrogel in time between 10 and 200 seconds, preferably between 20 and 100 seconds, more preferably between 30 and 60 seconds at a temperature between 15 to 38 °C.

The optimal gelation time found by the inventor allows the mixed polymer composition to be an "injectable composition".

The term "injectable composition" refers to a solution which is capable of forming a polymeric hydrogel once it has been injected. Therefore, said mixed polymer composition is fluid enough to enable injection of said mixed polymer composition. An injectable composition offers the advantages of in situ micro- and macro-gel formation at the injection site, such as the joint space, providing a good alignment with irregularly shaped defects and allowing an easy chondrocytes migration and growing into the biocompatible polymeric hydrogel and therefore a cartilage regeneration. Alternatively, the mixed polymer composition can be used to first generate microgels and after the microgels can be directly injected in the joint space.

The injection of the mixed polymer composition at the surgical site or to a site of an incision takes only few seconds. After injection few minutes are used to wait for the formation of the biocompatible polymeric hydrogel and the settlement of it. Moreover, the stability of the biocompatible polymeric hydrogel is checked by a soft movement of the area of application, for example by bending a knee if the biocompatible polymeric hydrogel was applied into the knee, followed by closing of the incision. This minimally invasive simple injection procedure represents a very advantageous technique compared to the traditional implantation surgery, commonly used in case of severe cartilage damage.

The injection of the mixed polymer composition at the surgical site or to a site of an incision may be performed by method known in the art. Preferably by using a needle or a cannula. When a needle is used, preferably the needle has a gauge between 12G and 23G, more preferably between 16G and 21G.

To allow the easy application of the mixed polymer composition of the current invention, the mixed polymer composition must have a favourable viscosity.

The inventor found that the initial viscosity of the mixed polymer composition at a fixed polymer concentration of 8.9 wt% (4.45 wt% of Dex-TA + 4.45 wt% of HA-TA) at T0 is between 15 and 25 mPa, preferably between 18 and 20 mPa.s at a shear rate of 10 Hz as determined in a rheometer in a in a double gap or cone-plate setup as required setup at 25t0.1 °C.

The term "T0" refers to the initial time at which tyramine-functionalized dextran (Dex-TA), tyramine-functionalized hyaluronic acid (HA-TA), peroxidase and hydrogen peroxide comes in contact with each other, before the gelation process starts. Therefore, the viscosity measurement at T0 is performed in absence of hydrogen peroxide avoiding the start of the gelation process and therefore of the formation of the biocompatible polymeric hydrogel of the invention. Once the gelation process will start, the viscosity of the mixed polymer composition will increase up to the formation of the biocompatible polymeric hydrogel.

The current invention also relates to a biocompatible polymeric hydrogel obtained by the reaction of a tyramine-functionalized dextran (Dex-TA) with a tyramine-functionalized hyaluronic acid (HA-TA) in the presence of peroxidase and hydrogen peroxide,

wherein the total weight ratio of Dex-TA and HA-TA is between 75:25 and 25:75, preferably between 60:40 and 40:60, more preferably between 55:45 and 45:55;

wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,

wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,

wherein the biocompatible polymeric hydrogel has a shear storage modulus between 10 and 100 kPa as measured in an oscillatory rheology setup, and shows a shrinkage $\alpha$ between -20% and 20%, wherein $\alpha$ is determined according to the formula (I):

$$\alpha = \frac{h_{mold} - h_{disc}}{h_{mold}} * 100\%$$

Formula (I)

wherein $\alpha$ is the shrinkage of the gel disc in %,
wherein $h_{mold}$ is the height of the mold, being 1.6 mm and
wherein $h_{disc}$ is the height of the gel disc in mm, determined under a normal force of 0.05 N in the oscillatory rheology setup.

The detailed description of the features as provided for the invention as described directly under the header "Detailed description of the features" equally applies to this preferred embodiment unless differently specified in the text below.

The shear storage modulus of the biocompatible polymeric hydrogel of the current invention has been evaluated in an oscillatory rheology setup. Samples for the oscillatory rheology measurements are prepared in 9 mm wide, 1.6 mm high molds to obtain cylindrical samples. After preparation, the cylindrical samples (gel discs) of the biocompatible polymeric hydrogel are incubated in phosphate-buffered saline overnight to reach equilibrium swelling. The gel disc is then taken out of the PBS, patted dry using a tissue paper to remove excess PBS and placed in oscillatory rheology setup with 8mm diameter spindle. The spindle is lowered onto the aligned gel disc, until a normal force of 0.05 ± 0.005 N is obtained. The measurement is then performed in oscillatory mode at frequency of 1 Hz with strain amplitude of 0.01% at 20 $\pm$ 0.1 °C.

The degradation of the biocompatible polymeric hydrogel of the current invention has been determined by incubating the hydrogels in hyaluronidase enzyme at 37°C for certain time duration. The biocompatible polymeric hydrogel has been prepared in 9 mm wide, 1.6 mm high molds to obtain cylindrical samples. The cylindrical samples (gel discs) of the biocompatible polymeric hydrogel is then incubated in phosphate-buffered saline overnight to reach equilibrium swelling. The biocompatible polymeric hydrogel is weighed after overnight equilibrium swelling. The phosphate-buffered saline is then removed, and the biocompatible polymeric hydrogel is incubated in 5 U/mL hyaluronidase solution at 37 °C. The degradation of the biocompatible polymeric hydrogel is then measured by weighing the biocompatible polymeric hydrogel at different time point and calculating the degradation as residual weight percentage of a biocompatible polymeric hydrogel that can be calculated as: wt/wi * 100% where, wt is the biocompatible polymeric hydrogel 's weight at the timepoint of interest and wi is the biocompatible polymeric hydrogel's initial weight after equilibrium swelling.

The inventor surprisingly found that the biocompatible polymeric hydrogel of the invention obtained by the reaction indicated above showed to be less susceptible to mechanical erosion between two moving joints.

The biocompatible polymeric hydrogel of the invention does not swell, and it has a good density allowing the chondrocytes to migrate and grow into biocompatible polymeric hydrogel of the invention.

The shear storage modulus that characterized the biocompatible polymeric hydrogel of the invention is between 10 and 100 kPa, preferably between 15 and 90 kPa, more preferably between 20 and 70 kPa, as measured in an oscillatory rheology setup, while its shrinkage $\alpha$ is between -20 % and 20 %, preferably between -15 % and 15 %, more preferably between -10 % and 10 %, as measured by Formula (I) as indicated above.

Also, the degradation time of the polymeric hydrogel of the invention is slower allowing for sufficient support in the defect so that more time is given to the chondrocytes to migrate and grow allowing for the cartilage regeneration.

The polymeric hydrogel of the invention is characterized by an in vitro degradation between 10 % and 50 %, preferably between 10 % and 40 %, more preferably between 10 % and 30 %, even more preferably between 10 % and 20 % after 30 days in hyaluronidase having a concentration of 5 Units/ml.

The term "Units/ml" in relation to the hyaluronidase refers to the concentration of hyaluronidase expressed in "active units" related to the enzymatic activity that will cause the same turbidity reduction as 1.0 unit of International Standard preparation.

The current invention relates also to the use of the biocompatible polymeric hydrogel of the invention as a medicament preventing or suppressing a joints pain condition in humans or in animals, wherein said pain condition is selected from the group consisting of: inflammatory pain, arthritis pain, osteoarthritis pain, osteochondritis dissecans pain, traumatic defects from trauma, accidents, slip or fall.

The term "used as a medicament for treating or preventing joints pain condition" as used herein refers to a treatment aiming to prevent or minimize joints pain due to damaged or missing tissue and wherein the provision of the polymeric hydrogel of the invention is aimed at improving regeneration of a damaged or missing tissue. Preferably, said damaged or missing tissue is cartilaginous tissue.

This pain condition may be due to disease as inflammation, arthritis, osteoarthritis or osteochondritis or it may be due to a traumatic defect from trauma, accidents, slip or fall.

Said polymeric hydrogel may also further comprise a medicament or drug which may be released following a sustained or extended-release mechanism. Hence, said biocompatible polymeric hydrogel may be used in a monotherapy or as an adjunct, or in combination with other known therapies.

The current invention relates also to a method of treating or preventing joints pain in humans or in animals comprising administering a therapeutical effective amount of the biocompatible polymeric hydrogel of the invention.

The therapeutical effective amount of the biocompatible polymeric hydrogel of the current invention may be administered to a surgical site or the site of an incision, injecting a therapeutical effective amount of the mixed polymer composition of the current invention at a time typically between 2 and 10 seconds.

It will be appreciated that the therapeutical effective amount of the biocompatible polymeric hydrogel or of the mixed polymer composition according to the invention required will be determined by for example the volume of said area where a tissue is to be repaired or replaced, but in addition to the swelling behavior and degradation characteristics of said polymeric hydrogel and whether the polymeric hydrogel is being used as a monotherapy or in a combined therapy. If said treatment is also used to provide a release of a medicament, then the amount is also dependent on the dose of a medicament incorporated in said polymeric hydrogel and the pharmacokinetics of said medicament and the advancement of the disease condition.

Known procedures, such as those conventionally employed by the pharmaceutical industry (e.g., in vivo experimentation, clinical trials, etc.), may be used to establish the therapeutical effective amount of the biocompatible polymeric hydrogel or of the mixed polymer composition according to the invention and the therapeutic regimes (such as doses and the frequency of administration).

The invention is now illustrated in a non-limiting manner by the following examples.

Examples

Abbreviations

Dex (Dextran), PNC (4-nitrophenyl chloroformate), LiCl (Lithium chloride), TA (tyramine), DMF (N,N-dimethylformamide), TA·HCl (tyramine hydrochloride), DMTMM (4-(4,6-dimethoxy-1,3,5- triazin-2-yl)-4-methylmorpholinium chloride), NaCl (Sodium chloride), HA (hyaluronic acid), PBS (phosphate-buffered saline or buffer solution with pH around 7.4), $H_2O_2$ (hydrogen peroxide), NMR (Nuclear magnetic resonance), wt% (weight percentage), Eq. (Molar equivalent).

Example 1

**Dex-TA synthesis**

Synthesis of dextran-p-nitrophenyl carbonate (Dextran-PNC)

LiCl (4.0 g, dried at 115 °C) and dextran (5.00 g, 30.8 mmol repeating units (r.u.)) are weighed into a 500 mL three necked round bottom flask equipped with a stirrer bar. The flask is evacuated and refilled with nitrogen 3 times, after which it is left under vacuum at 95 °C for 1.5h. After thoroughly drying, the flask was filled with nitrogen and 200 mL of anhydrous DMF was added via a cannula while stirring. The flask was then equipped with a thermometer and the mixture was heated to 95 °C while stirring the solution. Once the dextran was completely dissolved, the solution was cooled to 0 °C and anhydrous pyridine (2.0 ml, 25.8 mmol) was added. Subsequently, freshly sublimed para-nitrophenyl chloroformate (2.5 g, 12.4 mmol) was added in small portions, while keeping the temperature below 2 °C. After 1 hour, the reaction mixture was poured into 1 L of ice-cold ethanol. The precipitate was filtered off (Por 4) and washed with cold ethanol (3×100 mL) and subsequently with diethyl ether (3×100 mL). After drying under vacuum, the product was obtained as a white powder (6.00 g, 30.7 mmol r.u., 99 % yield, DS20%). $^1$H-NMR (400 MHz): $\delta$(ppm) = 3.0-4.0 (saccharide ring protons, m, 6H); 4.2-5.8 (anomeric and hydroxyl protons, m, 4H); 7.58 (Ar o-CH, d, 2H); 8.34 (Ar m-CH, d, 2H).

Synthesis of dextran-tyramine

Dextran-PNC (6.00 g, 30.7 mmol r.u., 6.15 mmol p-nitrophenyl carbonate) was weighed into a 250 mL three necked round bottom flask equipped with a stirrer bar. The flask was evacuated and refilled with nitrogen 3 times, after which the flask was filled with nitrogen and 100 mL of anhydrous DMF was added via a cannula while stirring. Once the dextran was completely dissolved, tyramine (1.69 g, 12.3 mmol) was added. After 1 hour, the reaction mixture was poured into 1 L of ice-cold ethanol. The precipitate was filtered off (Por 4) and washed with cold ethanol (3×100 mL) and subsequently with diethyl ether (3×100 mL). After drying under vacuum, the crude product was obtained as a white powder. The crude product was dissolved in 75 mL of Milli-Q water and dialysed against Milli-Q water for 3 days (MWCO 3500 Da), followed by filter sterilization and freeze-drying yielding the product as a white foam (5.04 g, 28.0 mmol, 92 % yield, DS10%). 1H-NMR (400 MHz): $\delta$(ppm) = 3.0-4.0 (saccharide ring protons, m, 6H); 4.8-5.2 (anomeric and hydroxyl protons, m, 4H); 6.7-7.1 (Ar m-CH, d, 2H); 6.9-7.3 (Ar o-CH, d, 2H).
The calculation of the DS of dextran-TA and dextran-PNC is based on the integrals of 4.8-5.2 ppm (corresponding to the 4 anomeric protons from dextran), compared with the integral of the aromatic protons of tyramine (6.7-7.1 and 6.9-7.3) or para-nitrophenyl (7.3-7.7 and 8.10-8.50). The DS of dextran is given as the percentage of saccharide units modified in dextran.

Example 2

**HA-TA synthesis**

Sodium hyaluronate (5.00 g, 12.5 mmol) was dissolved in 500 mL Milli-Q water (100 relative volume) in a 1 L round bottom flask equipped with a stirrer bar. While stirring at room temperature, DMTMM (3.46 g, 12.5 mmol, 1 molar equivalent) and TA·HCl (2.17 g, 12.5 mmol, 1 molar equivalent) were added subsequently. The addition of DMTMM and TA·HCl was repeated after 24 and 48 hours. After 72 hours, 40 mL NaCl (8 relative volume, saturated) was added to the reaction mixture and the reaction mixture was poured into 2.5 L (500 relative volume) cold ethanol. The crude product was isolated by centrifugation at 5000 rpm followed by drying in vacuo. The crude product was dissolved in 75 mL Milli-Q water and dialysed against Milli-Q water for 3 days (MWCO 1000 Da). Filter sterilization and lyophilization yielded the HA-TA product as a white foam (5.10 g, 12.4 mmol, 99 % yield, DS10%).
Successful HA-TA synthesis was confirmed using $^1$H NMR:

Baseline correction: splines method
Integrate peaks at 1.7-2.1 ppm, 6.7-7.1 ppm and 6.9-7.3 ppm.
Set peak area of peak at 1.7-2.1 ppm at 3000.
DS: add areas of peaks at 6.7-7.1 ppm and 6.9-7.3 ppm and divide by 40.
$^1$H-NMR (400 MHz, D2O): $\delta$(ppm) = 1.98 (acetyl-CH3, s, 3H); 2.75 (2-CH2, s, 2H); 2.90 (1-CH2, s, 2H); 3.2-4.2 (saccharide ring, m, 10H); 4.34 (s, 1H); 4.43 (d, 1H); 6.84 (Ar m-CH, d, 2H); 7.16 (Ar o-CH, d, 2H).

Example 3

**Hydrogel kit of parts suitable for preparing a biocompatible polymeric hydrogel preparation by a premix at 4:1 ratio**

Equal amount of Dex-TA and HA-TA are added to PBS (phosphate-buffered saline or buffer solution with pH around 7.4) to obtain polymer solution of concentration in a range of 11.0-13.0 wt%. HRP (horseradish peroxidase) is dissolved in PBS to obtain HRP of 30 U/mL (stock solution). Dilute 30 wt% $H_2O_2$ in PBS to obtain 0.25-0.5 wt% $H_2O_2$. Dex-TA/HA-TA polymer solution and HRP solution are premixed at 4:1 v/v ratio. Dex-TA/HA-TA polymer solution and $H_2O_2$ solution are also premixed at 4:1 v/v ratio. Subsequently both pre-mixed are combined in 1:1 v/v ratio.

Example 4

**Hydrogel kit of parts suitable for preparing a biocompatible polymeric hydrogel preparation by a premix at 7:1 ratio**

Equal amount of Dex-TA and HA-TA are added to PBS (phosphate-buffered saline or buffer solution with pH around 7.4) to obtain polymer solution of concentration in a range of 11.0-13.0 wt%. HRP (horseradish peroxidase) is dissolved in PBS to obtain HRP of 30 U/mL (stock solution). Dilute 30 wt% $H_2O_2$ in PBS to obtain 0.25-0.5 wt% $H_2O_2$. Dex-TA/HA-TA polymer solution and HRP solution are premixed at 7:1 ratio. This premix is combined with $H_2O_2$ in 4:1 ratio.

Methods

Several methods have been used to explore the properties, the structure, processability and performance of the mixed polymer composition and of a biocompatible polymeric hydrogel of the current invention.

Viscosity measurements

Viscosity measurements were carried out on a TA Instruments Discovery HR20 setup with a double gap geometry. During the viscosity measurement, the temperature was kept constant at 25°C, with a shear rate of 10 s-1 during 205 s, which was performed in duplicate. Viscosity values were calculated using software (Trios software, TA Instruments). The viscosities measured during the last 105 s of each measurement were averaged. The two average values for the duplicates were averaged to obtain the final viscosity. In this way the average dynamic (shear) viscosity of the biocompatible polymeric hydrogel is determined.

*A. Viscosity measuring geometry*

Double gap setup
Inside cup diameter: 40.0 mm
Inside bob diameter: 40.79 mm
Outside bob diameter: 43.88 mm
Outside cup diameter: 44.76 mm
Inner cylinder height: 55.0 mm
Immersed height: 53 mm
Operating gap: 2 mm
Bob material: aluminum
Peltier heating

*B. Measurement settings*
4 steps measurement have been performed.

1. Shear sweep (not used for data processing, only additional checks)

- T=25.0 ± 0.1 °C
- Shear sweep 0.01-100 Hz
- 10 points per decade
- 5s measurement per point

2. Time sweep (used for data processing)

- T=25.0 ± 0.1 °C

- 205 s measurement

- Shear rate = 10 Hz

- Sampling interval = 5s

3. Shear sweep (not used for data processing, only additional checks)

- T=25.0 ± 0.1 °C
- Shear sweep 0.01-100 Hz
- 10 points per decade
- 5s measurement per point

4. Time sweep (used for data processing)

- T=25.0 ± 0.1 °C

- 205 s measurement

- Shear rate = 10 Hz

- Sampling interval = 5s

*C. Viscosity data processing*

Only data acquired in steps 2 & 4 were used in data processing to acquire the solution's viscosity. Viscosity data acquired in the second half of step 2 (so the last 105 s) are averaged. Viscosity data acquired in the second half of step 4 (so the last 105 s) are averaged. The averages the last 105 s of steps 2 & 4 are averaged to obtain the viscosity of the solution.

Rheological measurement

1. Hydrogel disc preparation using mold for rheology

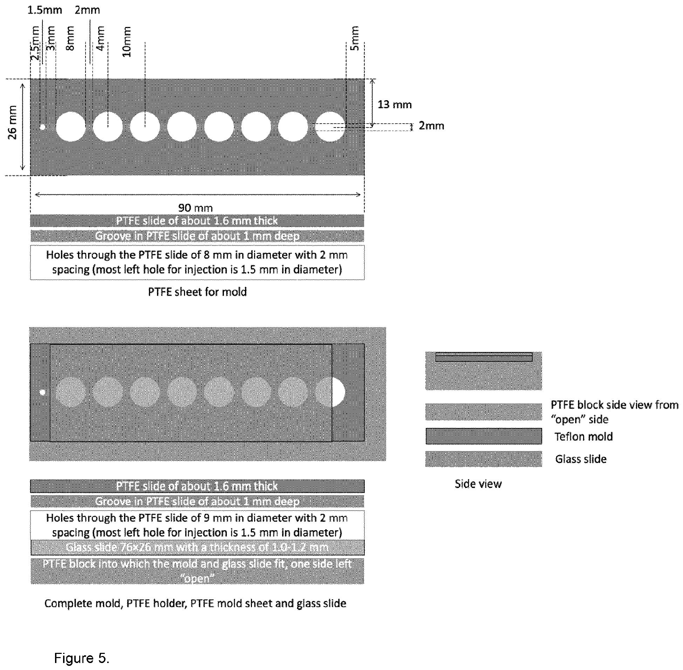

Hydrogel discs are prepared using the assembled mold shown in Figure 5.
The mold sheet is placed in the mold block, and a glass slide is placed on top and secured using binder clamps. Mixed components of the invention are injected through the 1.5 mm hole on the left side of the mold and allowed to gelate in the mold. Afterwards, the mold strip is removed from the mold block and the gel discs are pressed out of the mold strip. The gel discs are incubated in PBS at 2-8 °C for 12-168h before measurement.

2. Rheological measurement details

*A. Rheometry measuring geometry*

Parallel plates setup

Ø 8 mm spindle
Stainless steel
Peltier heating

*B. Rheometry measuring settings*
Spindle is lowered onto gel disc until a normal force (axial force) of 0.05 ± 0.005 N is achieved. Upon achieving the aforementioned normal force, the gap size is noted down.

*Measurement performed in oscillatory mode using the following settings:*

- Strain amplitude = 0.01 %
- Frequency = 1 Hz
- Measurement time = 120 s
- Sampling interval = 5 s
- Temperature = 20 ± 0.1 °C

*C. Rheometry data processing*
Storage modulus data over the last half (60-120s) of the measurement are averaged.

Swelling / shrinking

An oscillatory rheology setup (TA Instruments - Discovery HR20.) was used to determine the swelling/shrinking behavior of the biocompatible polymeric hydrogel.
The gel disc was prepared in 1.6 mm thick mold, the gel disc was placed in PBS at 2-7 °C for 12-168 h, dried and placed in oscillatory rheology setup with 8mm diameter spindle. The spindle was lowered onto the aligned gel disc, until a normal force of 0.05 ± 0.005 N was recorded.
The shrinkage $\alpha$ was determined according to the formula (I):

$$\alpha = \frac{h_{mold} - h_{disc}}{h_{mold}} * 100\%$$

Formula (I)

wherein $\alpha$ is the shrinkage of the gel disc in %,
wherein $h_{mold}$ is the height of the mold, being 1.6 mm and
wherein $h_{disc}$ is the height of the gel disc in mm, determined under a normal force of 0.05 N in the oscillatory rheology setup.

If the recorded height at a normal force of 0.05 N is for example 1.543 mm, this means that the biocompatible polymeric hydrogel has shrunk by 5.7% [(1.600-1.543)/1.600 *100%].

**Claims**

1. A kit of parts suitable for preparing a biocompatible polymeric hydrogel, the kit comprising at least 2 containers each containing an aqueous composition comprising at least one of tyramine-functionalized dextran (Dex-TA), tyramine-functionalized hyaluronic acid (HA-TA), peroxidase and hydrogen peroxide, which components -when mixed- allow the formation of the biocompatible polymeric hydrogel,

   wherein each of peroxidase and hydrogen peroxide are present in a different container,
   wherein the total weight ratio of Dex-TA and HA-TA is between 75:25 and 25:75, preferably between 60:40 and 40:60, more preferably between 55:45 and 45:55; wherein the concentration of Dex-TA ranges between 2 and 7 wt% relative to the sum of aqueous compositions of the kit of parts;
   wherein the concentration of HA-TA ranges between 2 and 7 wt% relative to the sum of aqueous compositions of the kit of parts;
   wherein the DEX-TA comprises unsubstituted and TA substituted repeat units of dextran, and wherein the amount of TA substituted repeat units of DEX-TA ($DS_{DEX}$) is between 5 mol% and 20 mol% relative to the total number of unsubstituted and TA substituted repeat units according to proton NMR;

wherein the HA-TA comprises unsubstituted and TA substituted repeat units of hyaluronic acid, and wherein the amount of TA substituted repeat units of HA-TA ($DS_{HA}$) is between 5 mol% and 20 mol % relative to the total number of unsubstituted and TA substituted repeat units according to proton NMR;

wherein the DEX-TA has a weight average molecular weight (Mw) between 15 and 650 kDa;

wherein the HA-TA has a weight average molecular weight (Mw) between 15 and 550 kDa;

wherein the peroxidase is present in a concentration between 0.25 and 10 Units/ml and the hydrogen peroxide is present in a concentration between 0.01 and 0.10 wt%, relative to the sum of aqueous compositions of the kit of parts.

2. A kit of parts according to claim 1, wherein a first container comprises an aqueous composition A, which composition A comprises

　　a. tyramine-functionalized dextran (Dex-TA),
　　b. tyramine-functionalized hyaluronic acid (HA-TA),
　　c. peroxidase,
　　and wherein a second container comprises an aqueous composition B, which composition B comprises
　　d. hydrogen peroxide and
　　e. optionally at least one of tyramine-functionalized dextran (Dex-TA) and tyramine-functionalized hyaluronic acid (HA-TA), preferably both Dex-TA and HA-TA,
　　wherein the total weight ratio of Dex-TA and HA-TA is between 75:25 and 25:75, preferably between 60:40 and 40:60, more preferably between 55:45 and 45:55;
　　wherein the concentration of Dex-TA ranges between 2 and 7 wt% relative to the sum of aqueous compositions of the kit of parts;
　　wherein the concentration of HA-TA ranges between 2 and 7 wt% relative to the sum of aqueous compositions of the kit of parts;
　　wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR;
　　wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR; wherein the DEX-TA has a weight average molecular weight (Mw) between 15 and 650 kDa;
　　wherein the HA-TA has a weight average molecular weight (Mw) between 15 and 550 kDa;
　　wherein the peroxidase is present in a concentration between 0.25 and 10 Units/ml and the hydrogen peroxide is present in a concentration between 0.01 and 0.10 wt%, relative to the sum of aqueous compositions of the kit of parts.

3. The kit of parts according to claim 1 or 2, wherein the peroxidase is selected from horseradish peroxidase (HRP), soybean peroxidase, myeloperoxidase, lactoperoxidase, Nitrospira sp. recombinant and Peroxidase and Lyngbya sp. recombinant lysyl oxidase.

4. The kit of parts according to claim 2 or 3, wherein the volume to volume ratio of composition A : composition B is in the range of 0.1 :1 to 10:1.

5. The kit of parts according to claims 2 to 4, wherein the components A and B are both fluids in a temperature range of 0°C to 37°C, having a viscosity between 1 mPa.s and 50 Pa.s, preferably between 1 and 100 mPa.s, more preferably below 50 mPa.s, even more preferably below 35 mPa.s as determined in a rheometer in a in a double gap or cone-plate setup as required at 25±0.1 °C at a shear rate of 10 Hz.

6. The kit of parts according to claim 1, wherein the aqueous composition of each container comprises between 80 and 99.9 wt% of water or of a water saline solution or of a phosphate-buffered solution, or of a phosphate-buffered saline solution.

7. The kit of parts according to claims 2 to 5, wherein each of composition A or composition B comprise between 80 and 99.9 wt% of water or of a water saline solution or of a phosphate-buffered solution, or of a phosphate-buffered saline solution.

8. A process for making a biocompatible polymeric hydrogel comprising the steps of:

a. mixing aqueous compositions comprising tyramine-functionalized dextran (Dex-TA), tyramine-functionalized hyaluronic acid (HA-TA), peroxidase and hydrogen peroxide, to obtain a mixed polymer composition,
b. allowing the mixed polymer composition to form the biocompatible polymeric hydrogel during a time between 10 and 200 seconds at a temperature between 15 to 38 °C,
wherein the total weight ratio of Dex-TA and HA-TA is between 75:25 and 25:75, preferably between 60:40 and 40:60, more preferably between 55:45 and 45:55; wherein the concentration of Dex-TA ranges between 2 and 7 wt% relative to the mixed polymer composition,
wherein the concentration of HA-TA ranges between 2 and 7 wt% relative to the mixed polymer composition,
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the DEX-TA has a weight average molecular weight (Mw) between 15 and 650 kDa,
wherein the HA-TA has a weight average molecular weight (Mw) between 15 and 550 kDa,
wherein the peroxidase is present in a concentration between 0.25 and 10 Units/ml, and the hydrogen peroxide is present in a concentration between 0.01 and 0.10 wt% relative to the mixed polymer composition.

9. A process for making a biocompatible polymeric hydrogel comprising the steps of:

a. mixing an aqueous composition A and an aqueous composition B obtaining a mixed polymer composition
b. allowing the mixed polymer composition to form the biocompatible polymeric hydrogel during a time between 10 and 200 seconds at a temperature between 15 to 38 °C,
wherein the aqueous composition A comprises tyramine-functionalized dextran (Dex-TA), tyramine-functionalized hyaluronic acid (HA-TA),peroxidase, and wherein the aqueous composition B comprises hydrogen peroxide and optionally at least one of tyramine-functionalized dextran (Dex-TA) and tyramine-functionalized hyaluronic acid (HA-TA), preferably both Dex-TA and HA-TA,
wherein the total weight ratio of Dex-TA and HA-TA is between 75:25 and 25:75, preferably between 60:40 and 40:60, more preferably between 55:45 and 45:55; wherein the concentration of Dex-TA ranges between 2 and 7 wt% relative to the sum of compositions A and B,
wherein the concentration of HA-TA ranges between 2 and 7 wt% relative to the sum of compositions A and B,
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the DEX-TA has a weight average molecular weight (Mw) between 15 and 650 kDa,
wherein the HA-TA has a weight average molecular weight (Mw) between 15 and 550 kDa,
wherein the peroxidase is present in a concentration between 0.25 and 10 Units/ml, and the hydrogen peroxide is present in a concentration between 0.01 and 0.10 wt% relative to the sum of aqueous compositions A and B.

10. A mixed polymer composition obtainable by mixing aqueous compositions comprising tyramine-functionalized dextran (Dex-TA), tyramine-functionalized hyaluronic acid (HA-TA), peroxidase and hydrogen peroxide,

wherein the total weight ratio of Dex-TA and HA-TA is between 75:25 and 25:75, preferably between 60:40 and 40:60, more preferably between 55:45 and 45:55; wherein the concentration of Dex-TA ranges between 2 and 7 wt% relative to the mixed polymer composition,
wherein the concentration of HA-TA ranges between 2 and 7 wt% relative to the mixed polymer composition,
wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the DEX-TA has a weight average molecular weight (Mw) between 15 and 650 kDa,
wherein the HA-TA has a weight average molecular weight (Mw) between 15 and 550 kDa,

wherein the peroxidase is present in a concentration between 0.25 and 10 Units/ml, and the hydrogen peroxide is present in a concentration between 0.01 and 0.10 wt% relative to the mixed polymer composition.

11. The mixed polymer composition according to claim 10, wherein the mixed polymer composition has a gelation time between 10 and 200 seconds at a temperature between 17 to 38 °C.

12. The mixed polymer composition according to claims 10 or 11, wherein the mixed polymer composition has a viscosity at T0 between 15 and 25 mPa.s at a shear rate of 10 Hz as determined in a rheometer in a in a double gap or cone-plate setup as required at 25t0.1 °C.

13. A biocompatible polymeric hydrogel obtained by the reaction of a tyramine-functionalized dextran (Dex-TA) with a tyramine-functionalized hyaluronic acid (HA-TA) in the presence of peroxidase and hydrogen peroxide,

wherein the total weight ratio of Dex-TA and HA-TA is between 75:25 and 25:75, preferably between 60:40 and 40:60, more preferably between 55:45 and 45:55; wherein the DEX-TA comprises unsubstituted and TA substituted repeat units of dextran, and wherein the amount of TA substituted repeat units of DEX-TA ($DS_{DEX}$) is between 5 mol% and 20 mol% according to proton NMR,
wherein the HA-TA comprises unsubstituted and TA substituted repeat units of hyaluronic acid, and wherein the amount of TA substituted repeat units of HA-TA ($DS_{HA}$) is between 5 mol% and 20 mol % according to proton NMR,
wherein the biocompatible polymeric hydrogel has a shear storage modulus between 10 and 100 kPa as measured in an oscillatory rheology setup, and shows a shrinkage $\alpha$ between -20 % and 20 %, wherein $\alpha$ is determined according to the formula (I):

$$\alpha = \frac{h_{mold} - h_{disc}}{h_{mold}} * 100\%$$

Formula (I)

wherein $\alpha$ is the shrinkage of the gel disc in %,
wherein $h_{mold}$ is the height of the mold, being 1.6 mm and
wherein $h_{disc}$ is the height of the gel disc in mm, determined under a normal force of 0.05 N in the oscillatory rheology setup.

14. The biocompatible polymeric hydrogel according to claim 13, wherein the biocompatible polymeric hydrogel has an in vitro degradation between 10 % and 50 % after 30 days in hyaluronidase having a concentration of 5 Units/ml.

15. The biocompatible polymeric hydrogel according to any one of claims 13 to 14 for use as a medicament preventing or suppressing a joints pain condition in humans or in animals, wherein said pain condition is selected from the group consisting of: inflammatory pain, arthritis pain, osteoarthritis pain, osteochondritis dissecans pain, traumatic defects from trauma, accidents, slip or fall.

Figure 1.

Figure 2.

Figure 3.

Figure 4.

PTFE sheet for mold

Holes through the PTFE slide of 8 mm in diameter with 2 mm
spacing (most left hole for injection is 1.5 mm in diameter)

Holes through the PTFE slide of 9 mm in diameter with 2 mm
spacing (most left hole for injection is 1.5 mm in diameter)

Complete mold, PTFE holder, PTFE mold sheet and glass slide

PTFE block side view from "open" side
Teflon mold
Glass slide

Side view

Figure 5.

## EUROPEAN SEARCH REPORT

Application Number

EP 23 15 2052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 132 201 B2 (KARPERIEN HERMANUS BERNARDUS JOHANNES [NL]; JIN RONG [NL] ET AL.) 15 September 2015 (2015-09-15) <br> * column 1, line 6 – line 13 * <br> * column 2, line 5 – line 32 * <br> * column 4, line 13 – line 31 * <br> * column 7, line 16 – line 50 * <br> ----- | 1-15 | INV. <br> A61L27/26 <br> A61L27/52 |
| X | JP 2018 114054 A (JSR CORP) 26 July 2018 (2018-07-26) <br> * paragraph [0006] – paragraph [0009] * <br> * paragraph [0012] * <br> * paragraph [0041] * <br> * paragraph [0045] – paragraph [0046] * <br> ----- | 1-15 | |
| A | RONG JIN ET AL: "Chondrogenesis in injectable enzymatically crosslinked heparin/dextran hydrogels", JOURNAL OF CONTROLLED RELEASE, ELSEVIER, AMSTERDAM, NL, vol. 152, no. 1, 25 January 2011 (2011-01-25), pages 186-195, XP028226339, ISSN: 0168-3659, DOI: 10.1016/J.JCONREL.2011.01.031 [retrieved on 2011-02-01] <br> * abstract * <br> ----- | 1-15 | TECHNICAL FIELDS SEARCHED (IPC) <br><br> A61L |

The present search report has been drawn up for all claims

| Place of search | Date of completion of the search | Examiner |
|---|---|---|
| The Hague | 29 June 2023 | Fort, Marianne |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.............................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 15 2052

This annex lists the patent family members relating to the patent documents cited in  the above-mentioned European search report.
The members are as contained in the European Patent Office EDP file on
The European Patent Office is in no way liable for these particulars which are merely given for the purpose of information.

29-06-2023

| Patent document cited in search report | | Publication date | Patent family member(s) | | Publication date |
|---|---|---|---|---|---|
| US 9132201 | B2 | 15-09-2015 | EP | 2498824 A2 | 19-09-2012 |
| | | | EP | 3067069 A1 | 14-09-2016 |
| | | | US | 2012276069 A1 | 01-11-2012 |
| | | | WO | 2011059326 A2 | 19-05-2011 |
| JP 2018114054 | A | 26-07-2018 | NONE | | |

For more details about this annex : see Official Journal of the European Patent Office, No. 12/82

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011059326 A

- WO 2019143247 A

**Non-patent literature cited in the description**

- **JIN R. et al.** *Biomaterials,* 2009, vol. 13, 2544-51
- **JIN R. et al.** *Biomaterials,* 2007, vol. 18, 2791-800

- **R JIN et al.** *Journal of Controlled Release,* 2008, vol. 132, e24-e6